# EUROPEAN PATENT APPLICATION

(11) **EP 4 417 185 A1**
(43) Date of publication of application: **21.08.2024**
(21) Application number: 22880797.0
(22) Date of filing: 29.09.2022
(51) Int. Cl.: A61K 8/37, A61K 8/06, A61K 8/27, A61K 8/29, A61K 8/36, A61K 8/39, A61K 8/85, A61Q 17/04

(54) **COSMETIC COMPOSITION**

(30) Priority: 13.10.2021 JP 2021168427
(71) Applicant: Shiseido Company, Ltd., Chuo-ku Tokyo 104-0061 (JP)
(72) Inventor: UJIMOTO, Kei, Tokyo 104-0061 (JP); SAKAE, Heini, Tokyo 104-0061 (JP)
(74) Representative: Cabinet Beau de Loménie
(86) International application number: PCT/JP2022/036518
(87) International publication number: WO 2023/063101

(57) **Abstract**

Provided is a cosmetic composition having further improved ultraviolet absorption ability in the entirety of the UVA and UVB regions.

The cosmetic composition is composed of components (i) to (iii) below, and an oil content which is capable of at least partially dissolving the component (i):
(i) a compound I having a structure of formula (I) below: 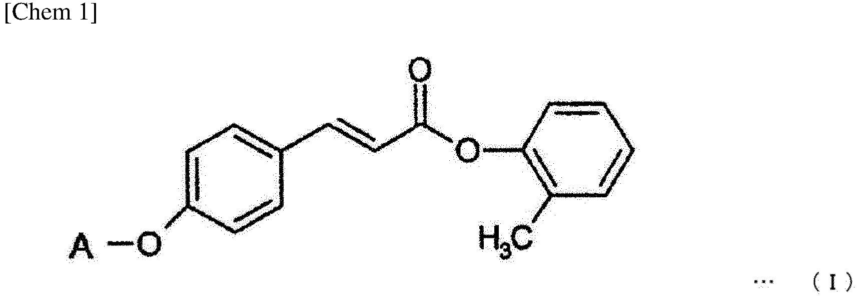 where -OA represents an alkoxy group,
(ii) ultraviolet scattering agent particles, and
(iii) a dispersant, wherein

a content of the ultraviolet scattering agent particles is more than 7.0% by mass, and
the dispersant contains at least one dispersant selected from the group consisting of polyhydroxystearic acid, isostearic acid, a polyglyceryl-based dispersant, and a sorbitan-based dispersant.

## Description

### FIELD

The present invention relates to a cosmetic composition.

### BACKGROUND

Protecting the skin from ultraviolet rays is an important issue in skin care and body care. In addition to medium-wavelength ultraviolet rays (UVB region: wavelength 290 to 320 nm), which are known to cause sunburn and inflammation, long-wavelength ultraviolet rays (UVA region: wavelength 320 to 400 nm) are known to have an effect on the skin (photoaging, etc.).

Conventional cosmetics for ultraviolet protection often include 2-ethylhexyl p-methoxycinnamate as an ultraviolet absorber. Though 2-ethylhexyl p-methoxycinnamate has the effect of absorbing ultraviolet rays in the UVB region, there is a need for an ultraviolet absorber having better ultraviolet absorption ability in the UVA region.

In connection thereto, the ultraviolet absorber disclosed in Patent Literature 1 reportedly has absorption capability in both the UVA region and the UVB region, has an ultraviolet suppression effect in a wide range of wavelengths, and has a previously unseen property: the ultraviolet absorption capability thereof increases in the UVA and UVB regions with the passage of time of ultraviolet irradiation.

More specifically, the ultraviolet absorber of Patent Literature 1 contains, as an active ingredient, a compound represented by general formula I: 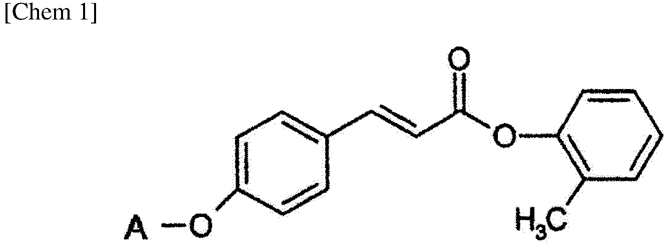 where -OA represents an alkoxy group.

### [CITATION LIST]

### [PATENT LITERATURE]

[PTL 1] WO 2009/041098

### SUMMARY

### [TECHNICAL PROBLEM]

However, regarding conventional cosmetics, which comprise a conventional ultraviolet absorbing agent (for example, octocrylene), and cosmetics in which the compound of Patent Literature 1 is used instead of this conventional ultraviolet absorbing agent, when the integrated values of absorbance in the range of 290 to 400 nm (the entirety of the UVA and UVB regions) thereof were compared, no major differences were found between these cosmetics.

Thus, the cosmetics including the ultraviolet absorbing agent of Patent Literature 1 still have room for improvement in terms of ultraviolet absorption ability in the entirety of the UVA and UVB regions.

The present invention aims to improve the situation described above, and an object thereof is to provide a cosmetic composition having further improved ultraviolet absorption ability in the entirety of the UVA and UVB regions.

### [SOLUTION TO PROBLEM]

The present invention, which can achieve the object described above, is as follows.

### <Aspect 1>

A cosmetic composition, comprising components (i) to (iii) below, and an oil content which is capable of at least partially dissolving the component (i):
(i) a compound I having a structure of formula (I) below: 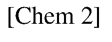 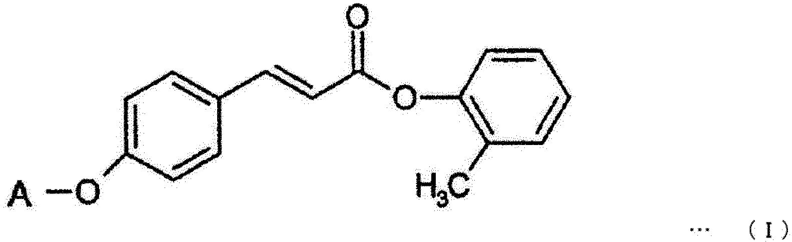 where -OA represents an alkoxy group,
(ii) ultraviolet scattering agent particles, and
(iii) a dispersant,

wherein a content of the ultraviolet scattering agent particles is more than 7.0% by mass, and
wherein the dispersant contains at least one dispersant selected from the group consisting of polyhydroxystearic acid, isostearic acid, a polyglycerol-based dispersant, and a sorbitan-based dispersant.

### <Aspect 2>

The composition according to Aspect 1, wherein the content of the ultraviolet scattering agent particles is 10% by mass or more.

### <Aspect 3>

The composition according to Aspect 1 or 2, wherein the dispersant contains at least one dispersant selected from the group consisting of the polyhydroxystearic acid and the polyglycerol-based dispersant.

### <Aspect 4>

The composition according to any one of Aspects 1 to 3, wherein the polyglyceryl-based dispersant is a polyglycerin-modified silicone or a fatty acid polyglyceryl ester having 3 to 8 glycerin units.

### <Aspect 5>

The composition according to any one of Aspects 1 to 4, wherein a content of the dispersant is 0.5% by mass or more and 3.5% by mass or less.

### <Aspect 6>

The composition according to any one of Aspects 1 to 5, wherein the oil content contains a polar oil.

### <Aspect 7>

The composition according to any one of Aspects 1 to 6, which is an oil-in-water cosmetic.

### <Aspect 8>

The composition according to any one of Aspects 1 to 6, which is a water-in-oil cosmetic.

### <Aspect 9>

The composition according to any one of Aspects 1 to 6, which is an oil-based cosmetic.

### <Aspect 10>

The composition according to any one of Aspects 1 to 9, which is a sunscreen cosmetic composition.

### [ADVANTAGEOUS EFFECTS OF INVENTION]

According to the present invention, there can be provided a cosmetic composition having further improved ultraviolet absorption ability in the entirety of the UVA and UVB regions.

### DESCRIPTION OF EMBODIMENTS

The embodiments of the present invention will be described in detail below. Note that the present invention is not limited to the following embodiments, and various changes can be made within the scope of the spirit of the invention.

### <<Cosmetic Composition>>

The cosmetic composition of the present invention (hereinafter also referred to simply as "the composition of the present invention") is:
a cosmetic composition, comprising components (i) to (iii) below, and an oil content which is capable of at least partially dissolving the component (i):
   (i) a compound I having a structure of formula (I) below: 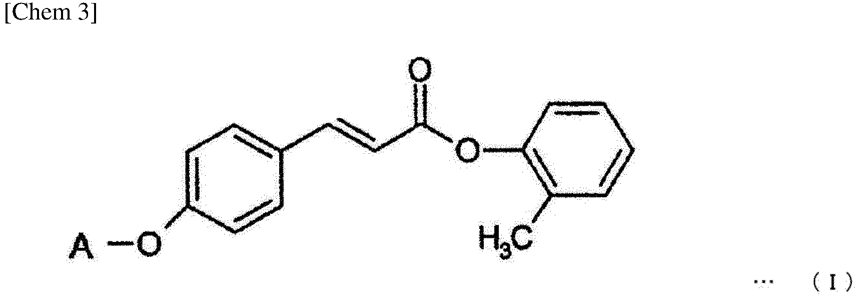 where -OA represents an alkoxy group,
   (ii) ultraviolet scattering agent particles, and
   (iii) a dispersant,
wherein a content of the ultraviolet scattering agent particles is more than 7.0% by mass, and
wherein the dispersant contains at least one dispersant selected from the group consisting of polyhydroxystearic acid, isostearic acid, a polyglyceryl-based dispersant, and a sorbitan-based dispersant.

As a result of rigorous investigation, the present inventors have unexpectedly discovered that when a specific dispersant is contained and the amount of the ultraviolet scattering agent particles in the cosmetic is relatively high (for example, more than 7.0% by mass), by using the compound I having the structure of formula (I) above, as an ultraviolet absorber in place of a conventional ultraviolet absorber, the integrated value of absorbance at 290 to 400 nm (i.e., the ultraviolet absorption ability in the entirety of the UVA and UVB regions) is significantly improved.

### <Component (i): Compound I>

The compound I according to the present invention has a structure of the following formula (I): 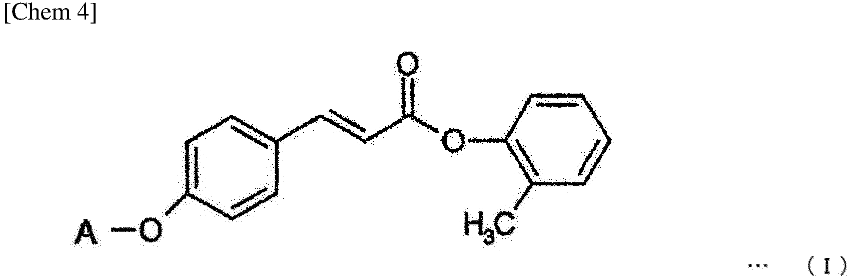 where -OA represents an alkoxy group.

In formula (I), -OA represents an alkoxy group, and more specific examples thereof include, but are not limited to, a methoxy group and an ethoxy group.

In the present invention, the compound I may have the same structure as the compound represented by general formula I disclosed in Patent Literature 1.

Furthermore, in the composition of the present invention, from the viewpoint of further exhibiting the effects of the present invention, it is preferable that the compound I be at least partially in a dissolved state, in particular 50% by mass or more thereof be in a dissolved state, more particularly 90% by mass or more thereof be in a dissolved state, further particularly 99% by mass or more thereof be in a dissolved state, and most particularly 100% by mass thereof be in a dissolved state.

In the composition of the present invention, the content of the compound I is not particularly limited, and may be, relative to the entire cosmetic composition, for example, 0.01% by mass or more, 0.05% by mass or more, 0.1% by mass or more, 0.5% by mass or more, 1.0% by mass or more, 1.5% by mass or more, 2.0% by mass or more, 2.5% by mass or more, 3.0% by mass or more, 3.5% by mass or more, 4.0% by mass or more, 4.5% by mass or more, 5.0% by mass or more, 5.5% by mass or more, 6.0% by mass or more, 6.5% by mass or more, 7.0% by mass or more, 7.5% by mass or more, 8.0% by mass or more, 8.5% by mass or more, 9.0% by mass or more, 9.5% by mass or more, or 10% by mass or more, and may be 50% by mass or less, 40% by mass or less, 30% by mass or less, 20% by mass or less, 10% by mass or less, 8.0% by mass or less, 5.0% by mass or less, or 2.0% by mass or less.

### <Component (ii): Ultraviolet Scattering Agent Particles>

By containing ultraviolet scattering agent particles in the composition of the present invention, the ultraviolet protection effect can be enhanced. In the present invention, the ultraviolet scattering agent particles are not particularly limited, and may be particles of at least one metal oxide selected from the group consisting of titanium oxide, zinc oxide, iron oxide, and cerium oxide.

Furthermore, the ultraviolet scattering agent particles may be subjected to a hydrophobization treatment. The hydrophobization treatment may be performed using a known surface treatment agent for hydrophobization, and examples thereof include a fatty acid treatment, fluorine compound treatment, silicone treatment, silicone resin treatment, pendant treatment, silane coupling agent treatment, titanium coupling agent treatment, oil treatment, N-acylated lysine treatment, polyacrylic acid treatment, amino acid treatment, inorganic compound treatment, plasma treatment, mechanochemical treatment, silane compound treatment, and silazane compound treatment. Among these treatments, from the viewpoint of dispersion stability, a fatty acid treatment with dextrin palmitate or aluminum stearate, or a silicone treatment is preferable.

The average primary particle diameter of the ultraviolet scattering agent particles is not particularly limited, and may be, for example, 5 nm or more, 10 nm or more, or 15 nm or more, and may be 200 nm or less, 100 nm or less, or 50 nm or less. Note that the "average primary particle diameter" may be determined as the circle equivalent diameter of the projected area of the primary particles in an SEM image.

The shape of the ultraviolet scattering agent particles is not particularly limited, and examples thereof include spherical, plate-like, rod-like, spindle-like, needle-like, and irregular shapes.

In the composition of the present invention, the content of ultraviolet scattering agent particles is more than 7.0% by mass relative to the entire cosmetic composition, and more specifically, may be, for example, more than 7.0% by mass, 7.5% by mass or more, 8.0% by mass or more, 8.5% by mass or more, 9.0% by mass or more, 10% by mass or more, 11% by mass or more, 12% by mass or more, 13% by mass or more, 14% by mass or more, or 15% by mass or more. Furthermore, the upper limit of the content of the ultraviolet scattering agent particles is not particularly limited, and may be, relative to the entire cosmetic composition, for example, 30% by mass or less, 20% by mass or less, or 18% by mass or less.

### <Component (iii): Dispersant>

In the composition of the present invention, the dispersant can uniformly disperse ultraviolet scattering agent particles in a medium (for example, oil content).

Further, the present inventors have unexpectedly discovered that by using the specific dispersant described below as the dispersant for dispersing the ultraviolet scattering agent particles, the integrated value of the absorbance in the wavelength range of 290 to 400 nm of the composition is further significantly improved.

In the present invention, from the viewpoint of further exhibiting the effects of the present invention, for example, the dispersant preferably contains at least one dispersant selected from the group consisting of polyhydroxystearic acid, isostearic acid, a polyglyceryl-based dispersant, and a sorbitan-based dispersant, more preferably contains at least one dispersant selected from the group consisting of polyhydroxystearic acid and a polyglyceryl-based dispersant, and particularly preferably contains polyhydroxystearic acid. Note that in the present invention, the specific dispersant described above is also referred to as the "specific dispersant of the present invention."

### (Hydroxypolystearic Acid)

In the present invention, a compound obtained by oligomerizing hydroxystearic acid by forming an ester bond can be used as the polyhydroxystearic acid, and a commercially available product may also be used. Furthermore, the degree of polymerization of the polyhydroxystearic acid is not particularly limited, and may be, for example, 4 to 8.

### (Polyglyceryl-based Dispersant)

In the present invention, the polyglyceryl-based dispersant may be a polyglycerin-modified silicone or a fatty acid polyglyceryl ester having 3 to 8 glycerin units.

In the present invention, the polyglycerin-modified silicone is represented by the following formula (II):

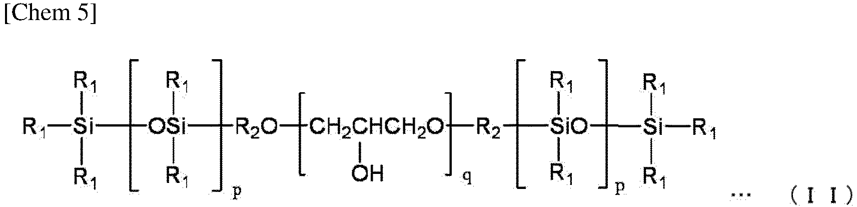

In formula (II), R₁ represents a linear or branched alkyl group having 1 to 12 carbon atoms, or a phenyl group; R₂ represents an alkylene group having 2 to 11 carbon atoms; p is a number of 10 to 120; and q is a number from 1 to 11.

The polyglycerin-modified silicone represented by the above formula (II) can be obtained by, for example, adding a chloroplatinic acid isopropyl alcohol solution to a mixed solution of polyglycerin diallyl ether and a single-end hydrogenated dimethylpolysiloxane, heating and reacting, adding an aqueous solution of hydrochloric acid thereto, performing overheating hydrolysis, neutralizing by adding aqueous sodium bicarbonate, purifying, and then evaporating. Note that the polyglycerin-modified silicone according to the present invention is not limited to this production method.

In the present invention, examples of the polyglycerin-modified silicone include, but are not limited to, bisbutyl dimethicone polyglyceryl-3, which is defined as a cosmetic labeling name.

Furthermore, the polyglycerin-modified silicones represented by the above formula (II) may be commercially available products.

In the present invention, examples of the fatty acid polyglyceryl ester having 3 to 8 glycerin units include esters of polyglycerin and branched or linear fatty acids.

The fatty acid constituting the fatty acid polyglycerin ester may be selected from monohydric (monohydroxy) fatty acids having 12 to 22 carbon atoms. For example, saturated monohydric fatty acids such as isostearic acid and unsaturated monohydric fatty acids such as ricinoleic acid are preferable, and among them, polymers of ricinoleic acid are particularly preferable.

In the present invention, specific examples of the fatty acid polyglyceryl ester having 3 to 8 glycerin units include, but are not limited to, polyglyceryl-6 polyricinoleate.

### (Sorbitan-based Dispersant)

In the present invention, examples of the sorbitan-based dispersant include sorbitan fatty acid esters. More specifically, examples thereof include, but are not limited to, sorbitan monolaurate, sorbitan monopalmitate, sorbitan monostearate, sorbitan isostearate, sorbitan sesquiisostearate, sorbitan monooleate, sorbitan sesquioleate, and sorbitan trioleate.

In the composition of the present invention, the content of the dispersant is not particularly limited, and may be, relative to the entire cosmetic composition, for example, 0.5% by mass or more, 0.6% by mass or more, 0.7% by mass or more, 0.8% by mass or more, 0.9% by mass or more, 1.0% by mass or more, 1.1% by mass or more, 1.2% by mass or more, 1.3% by mass or more, 1.4 % by mass or more, 1.5% by mass or more, 1.6% by mass or more, 1.7% by mass or more, 1.8% by mass or more, 1.9% by mass or more, or 2.0% by mass or more, and may be 3.5% by mass or less, 3.2% by mass or less, 3.0% by mass or less, 2.8% by mass or less, 2.5% by mass or less, 2.2% by mass or less, or 2.0% by mass or less.

### <Oil Content>

The composition of the present invention contains an oil content. The oil content contained in the composition of the present invention is an oil content that can at least partially dissolve the compound I described above, and thus, the compound I described above can be at least partially dissolved therein.

In the composition of the present invention, the content of the oil content is not particularly limited, and may be, for example, 5.0% by mass or more, 10% by mass or more, 15% by mass or more, 20% by mass or more, 25% by mass or more, 30% by mass or more, 35% by mass or more, 40% by mass or more, or 50% by mass or more, and 99.9% by mass or less, 90% by mass or less, 80% by mass or less, 70% by mass or less, 60% by mass or less, 55% by mass or less, 50% by mass or less, 45% by mass or less, 40% by mass or less, 35% by mass or less, or 30% by mass or less.

In the present invention, the oil content is not particularly limited as long as the compound I described above can be at least partially dissolved therein. More specifically, the oil content may contain one or more of, for example, a polar oil, silicone oil, or hydrocarbon oil. From the viewpoint of dissolution of the compound I described above, the oil content preferably contains a polar oil.

### (Polar Oil)

"Polar oil" as used herein refers to polar oils with high polarity among oils usable in cosmetics, other than silicone oils, and refers to those having an IOB value of, for example, 0.10 or more, 0.11 or more, 0.12 or more, or 0.13 or more. Furthermore, the IOB value of the polar oil according to the present invention may be 0.50 or less, 0.45 or less, or 0.40 or less. The IOB value is an abbreviation of Inorganic/Organic Balance, is a value representing the ratio of the inorganic value to the organic value, and is an index indicating the degree of polarity of the organic compound. Specifically, the IOB value is expressed as IOB value = inorganic value / organic value. Regarding the "inorganic value" and the "organic value," depending on the type of atom or functional group, the "inorganic value" or "organic value" is set in a molecule, for example, one carbon atom has an "organic value" of 20 and one hydroxyl group has an "inorganic value" of 100, and by integrating the "inorganic value" or "organic value" of each atom and functional group in an organic compound, the IOB value of the organic compound can be calculated (for example, refer to Yoshio KODA, "Organic Conceptual Diagram - Basics and Applications," pp. 11-17, published by Sankyo Publishing, 1984).

Note that in the present invention, oil contents having an IOB value of 0.10 or more for use as the ultraviolet absorber are excluded from the "polar oils" herein.

In the present invention, the polar oil may be, for example, at least one selected from the group consisting of ester oils, ether oils, higher alcohols, and fatty acids having an IOB value of 0.10 or more.

More specifically, the polar oil may be, for example, at least one selected from the group consisting of bisethoxydiglycol cyclohexane-1,4-dicarboxylate (IOB value 1.03), phenoxyethyl caprylate (IOB value 0.29), PPG-30-BUTETH-30 (IOB value 0.94), diisopropyl sebacate (IOB value 0.4), neopentyl glycol diheptanoate (IOB value 0.33), diethylhexyl succinate (IOB value 0.32), isononyl isononanoate (IOB value 0.2), isopropyl myristate (IOB value = 0.18), octyl palmitate (IOB value = 0.13), isopropyl palmitate (IOB value = 0.16), butyl stearate (IOB value = 0.14), hexyl laurate (IOB value = 0.17), myristyl myristate (IOB value = 0.11), decyl oleate (IOB value = 0.11), isotridecyl isononanoate (IOB value = 0.15), cetyl ethylhexanoate (IOB value = 0.13), pentaerythrityl tetraethylhexanoate (IOB value = 0.35), dioctyl succinate (IOB value = 0.36), glycol distearate (IOB value = 0.16), glyceryl diisostearate (IOB value = 0.29), neopentyl glycol dicaprate (IOB value = 0.25), diisostearyl malate (IOB value = 0.28), trimethylolpropane triisostearate (IOB value = 0.16), glyceryl tri-2-ethylhexanoate (triethylhexanoin) (IOB value = 0.35), trimethylolpropane trioctanoate (IOB value = 0.33), trimethylolpropane triisostearate (IOB value = 0.16), diisobutyl adipate (IOB value = 0.46), N-lauroyl-L-glutamic acid-2-octyldodecyl ester (IOB value = 0.29), 2-hexyldecyl adipate (IOB value = 0.16), ethylhexyl methoxycinnamate (IOB value = 0.28), 2-ethylhexyl palmitate (IOB value = 0.13), 2-ethylhexyl ethylhexanoate (IOB value = 0.2), triisostearin (IOB value = 0.16), PPG-3 dipivalate (IOB value = 0.52), and tri(caprylic acid/capric acid) glyceryl (IOB value = 0.33), but it is not limited thereto.

In the present invention, the amount of the polar oil contained in the oil content is not particularly limited, and may be, relative to a total of 100 parts by mass of the oil content, for example, 5.0 parts by mass or more, 10 parts by mass or more, 15 parts by mass or more, or 20 parts by mass or more, and may be 90 parts by mass or less, 80 parts by mass or less, 70 parts by mass or less, 60 parts by mass or less, 50 parts by mass or less, 40 parts by mass or less, 30 parts by mass or less, or 20 parts by mass or less.

Furthermore, when the oil content of the composition of the present invention contains a polar oil, the polar oil and the compound I described above may be included at the following composition ratio. More specifically, relative to a total of 100 parts by mass of the polar oil and the compound I , the compound I may be 0.01 parts by mass or more, 0.05 parts by mass or more, 0.1 parts by mass or more, 0.5 parts by mass or more, 1.0 part by mass or more, 1.5 parts by mass or more, 2.0 parts by mass or more, 2.5 parts by mass or more, 3.0 parts by mass or more, 3.5 parts by mass or more, 4.0 parts by mass or more, 4.5 parts by mass or more, 5.0 parts by mass or more, 5.5 parts by mass or more, 6.0 parts by mass or more, 6.5 parts by mass or more, 7.0 parts by mass or more, 7.5 parts by mass or more, 8.0 parts by mass or more, 8.5 parts by mass or more, 9.0 parts by mass or more, 9.5 parts by mass or more, 10 parts by mass or more, 15 parts by mass or more, or 20 parts by mass or more, and may be 50 parts by mass or less, 45 parts by mass or less, 40 parts by mass or less, 35 parts by mass or less, 30 parts by mass or less, 25 parts by mass or less, 20 parts by mass or less, 15 parts by mass or less, or 10 parts by mass or less.

### (Silicone Oil)

In the present invention, "silicone oil" refers to oil contents having a main skeleton formed by siloxane bonds among those usable in cosmetics.

In the present invention, the silicone oil may be a volatile silicone oil or a nonvolatile silicone oil.

In the present invention, the boiling point under one atmosphere (101.325 kPa) can be used as a guideline for "volatile." The boiling point may be, for example, 250 °C or lower, 240 °C or lower, or 230 °C or lower, and may be 80 °C or higher, 100 °C or higher, 120 °C or higher, 150 °C or higher, or 160 °C or higher. Furthermore, in the present disclosure, "nonvolatile" is intended to mean that the volatile content is 5% or less when a sample is placed in a sufficiently large flat-bottomed dish and allowed to stand at 105 °C for 3 hours.

Furthermore, in the present invention, the silicone oil may be an acyclic silicone oil (i.e., a linear silicone oil) or a cyclic silicone oil.

Specific examples of the silicone oil include, but are not limited to, acyclic silicone oils such as polydimethylsiloxane (dimethicone), trisiloxane, caprylylmethicone, methylphenylpolysiloxane, and methylhydrogenpolysiloxane, cyclic silicone oils such as octamethylcyclotetrasiloxane, decamethylcyclopentasiloxane, and dodecamethylcyclohexasiloxane, and mixtures of two or more of these. Furthermore, from the viewpoint of feeling during use, an acyclic silicone is particularly preferable as the silicone oil.

In the present invention, the amount of the silicone oil contained in the oil content is not particularly limited, and may be, relative to a total of 100 parts by mass of the oil content, for example, 5.0 parts by mass or more, 10 parts by mass or more, 15 parts by mass or more, or 20 parts by mass or more, and may be 90 parts by mass or less, 80 parts by mass or less, 70 parts by mass or less, 60 parts by mass or less, 50 parts by mass or less, 40 parts by mass or less, 30 parts by mass or less, or 20 parts by mass or less.

### (Hydrocarbon Oil)

In the present invention, "hydrocarbon oil" refers to a hydrocarbon oils other than the polar oils described above.

The hydrocarbon oil may be a volatile hydrocarbon oil or a nonvolatile hydrocarbon oil.

Specific examples of the hydrocarbon oil include, but are not limited to, decane, dodecane, isododecane, isohexadecane, liquid paraffin, squalane, squalene, paraffin, and mixtures of two or more thereof.

### <Applications and Forms of Composition of the Present Invention>

The composition of the present invention can be suitably used as a cosmetic or a raw material thereof. Thus, in particular, the present invention provides a sunscreen cosmetic composition.

The form of the composition of the present invention is not particularly limited, and may be, for example, an oil-in-water emulsified cosmetic composition, a water-in-oil emulsified cosmetic composition, or an oil-based cosmetic composition.

### <Other Components>

The cosmetic composition of the present invention may further contain, in addition to the above-mentioned components, arbitrary aqueous components or other components that can be used in the cosmetics field. As the other components, for example, other components such as other ultraviolet absorbers, surfactants, moisturizers, sequestering agents, natural and synthetic polymers, water-soluble and oil-soluble polymers, various extracts, colorants such as organic dyes, preservatives, antioxidants, pigments, thickeners, pH adjusters, fragrances, cooling agents, antiperspirants, disinfectants, skin activators, other chemicals, and various powders, may be further contained, but the other components are not limited thereto.

### (Other Ultraviolet Absorbers)

In the composition of the present invention, the ultraviolet absorber may further contain an ultraviolet absorber other than the compound I described above.

In the present invention, examples of an ultraviolet absorber other than the compound I include, but are not limited to, benzoic acid derivatives, salicylic acid derivatives, cinnamic acid derivatives, dibenzoylmethane derivatives, β,β-diphenylacrylate derivatives, benzophenone derivatives, benzylidene camphor derivatives, phenylbenzimidazole derivatives, triazine derivatives, phenylbenzotriazole derivatives, anthranyl derivatives, imidazoline derivatives, benzalmalonate derivatives, and 4,4-diarylbutadiene derivatives.

### (Surfactant)

In the present invention, the surfactant may function as an emulsifier, or may have function as both an emulsifier and a dispersant.

The surfactant is not particularly limited, and examples thereof include, but are not limited to, PEG-10 dimethicone, PEG-9 polydimethylsiloxyethyl dimethicone, and lauryl PEG-9 polydimethylsiloxyethyl dimethicone.

In the composition of the present invention, when a surfactant is contained, the content thereof is not particularly limited, and is, for example, 1.0% by mass or more and 10% by mass or less.

### (Water-Soluble Component)

The composition of the present invention may further contain a water-soluble component such as a lower alcohol. Examples of the lower alcohol include, but are not limited to, ethanol, propanol, butanol, pentanol, and hexanol.

Further, when a water-soluble component is contained, the content thereof is not particularly limited, and may be, for example, 1.0 to 10% by mass.

### EXAMPLES

The present invention will be described in more detail below with reference to Examples, but the present invention is not limited thereto.

### <<Preparation of Water-in-Oil Cosmetic Compositions

### <Reference Examples 1 to 3, Comparative Examples 1 to 4, and Examples 1 to 4>

The compositions of Reference Examples 1 to 3, Comparative Examples 1 to 4, and Examples 1 to 4 were prepared based on the compositions (% by mass) of Table 1 below.

Note that in Reference Examples 1 and 3, Examples 1 to 4, and Examples 5 to 20 described below, the compound represented by the following structural formula was used as the compound I:

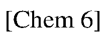

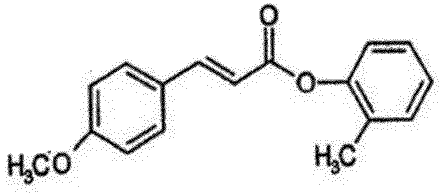

The ultraviolet absorption wavelength of each prepared composition was measured, and the integrated value of absorbance at 290 to 400 nm (the entirety of the UVA and UVB regions) was determined. As shown in Table 1, the ultraviolet absorption ability improvement effect of the composition of each Example was determined as a "boost rate" from the corresponding Example serving as a "boost rate calculation reference" (hereinafter, the boost rates of the other Examples and Comparative Examples were also determined using the same method). Note that the boost rate was calculated based on the following formula: Boost rate = (integrated value of absorbance in Example to be determined / integrated value of absorbance in the Example used as reference for calculation of boost rate) × 100%

For example, the boost rate of Reference Example 1 was calculated using Reference Example 2 as a calculation reference. More specifically, the boost rate of Reference Example 1 = (integrated value of absorbance of Reference Example 1 / integrated value of absorbance of Reference Example 2) × 100%).

**[Table 1]**

| (Table 1: Reference Examples 1 to 3, Comparative Examples 1 to 4, and Examples 1 to 4) | | | | | | | | | | | | |
|---|---|---|---|---|---|---|---|---|---|---|---|---|
| Composition | | Reference Example 1 | Reference Example 2 | Reference Example 3 | Comparative Example 1 | Comparative Example 2 | Comparative Example 3 | Comparative Example 4 | Example 1 | Example 2 | Example 3 | Example 4 |
| Water | Water | 44.7 | 44.7 | 43.7 | 36.7 | 36.7 | 33.7 | 33.7 | 33.7 | 33.7 | 28.7 | 28.7 |
| Specific dispersant of present invention | Isostearic acid | - | - | - | - | 1.0 | - | 1.0 | - | 1.0 | - | 1.0 |
| | Polyhydroxystearic acid | - | - | 1.0 | 1.0 | - | 1.0 | - | 1.0 | - | 1.0 | - |
| Another dispersant | PEG-9 polydimethylsiloxyethyl dimethicone | 2.0 | 2.0 | 2.0 | 2.0 | 2.0 | 2.0 | 2.0 | 2.0 | 2.0 | 2.0 | 2.0 |
| UV absorber | Compound I | 1.0 | - | 1.0 | 1.0 | 1.0 | - | - | 1.0 | 1.0 | 1.0 | 1.0 |
| | Octocrylene | - | 1.0 | - | - | - | 1.0 | 1.0 | - | - | - | - |
| UV scattering agent particles | Fatty acid-treated titanium oxide | - | - | - | 4.0 | 4.0 | 5.0 | 5.0 | 5.0 | 5.0 | 10.0 | 10.0 |
| | Fatty acid-treated zinc oxide | - | - | - | 3.0 | 3.0 | 5.0 | 5.0 | 5.0 | 5.0 | 5.0 | 5.0 |
| Others | Others | 54.3 | 54.3 | 54.3 | 54.3 | 54.3 | 54.3 | 54.3 | 54.3 | 54.3 | 54.3 | 54.3 |
| Total | | 100 | 100 | 100 | 100 | 100 | 100 | 100 | 100 | 100 | 100 | 100 |
| Evaluation | Boost rate | 100% | - | 100% | 225% | 215% | 325% | 310% | 430% | 370% | 520% | 450% |
| | Boost rate calculation reference | Reference Example 2 | - | Reference Example 1 | | | | | | | | |

The details of components of "others" of Table 1 are as follows:

| | |
|---|---|
| ·Diisopropyl sebacate (oil content) | 10% by mass |
| ·Triethylhexanoin (oil content) | 10% by mass |
| ·Isododecane (oil content) | 10% by mass |
| ·Dimethicone (oil content) | 10% by mass |
| ·Ethanol | 5.0% by mass |
| ·Glycerin | 1.0% by mass |
| ·Disteardimonium hectorite | 0.5% by mass |
| ·Silica powder | 5.0% by mass |
| ·Phenoxyethanol | 0.5% by mass |
| ·Na chloride | 0.1% by mass |
| ·EDTA-3Na | 0.2% by mass |

As is clear from Table 1, when the composition of Reference Example 2, which used octocrylene as the ultraviolet absorber, was used as the reference, the boost rate of the composition of Reference Example 1, in which the compound I was used instead of octocrylene, was 100%. From this, it is understood that the ultraviolet absorption ability of the composition of Reference Example 1 and the ultraviolet absorption ability of the composition of Reference Example 2 are comparable to each other, i.e., when the ultraviolet scattering agent particles are not contained, even if another dispersant (PEG-9 polydimethylsiloxyethyl dimethicone) is contained, regarding the integrated value of absorbance in the entirety of the UVA and UVB regions, the ultraviolet absorption ability of the compound I is comparable to the ultraviolet absorption ability of octocrylene, which is a conventional ultraviolet absorber.

Furthermore, when the composition of Reference Example 1 was used as a reference, the boost rate of the composition of Reference Example 3, which used polyhydroxystearic acid, which was one of the specific dispersants of the present invention, as a dispersant, was 100%. From this, it is understood that the ultraviolet absorption ability of the composition of Reference Example 3 and the ultraviolet absorption ability of the composition of Reference Example 1 are comparative to each other, i.e., when ultraviolet scattering agent particles are not contained, even if the specific dispersant of the present invention is further contained, regarding the integrated value of absorbance in the entirety of the UVA and UVB regions, the ultraviolet absorption ability of the compound I is comparable to the ultraviolet absorption ability of octocrylene, which is a conventional ultraviolet absorber.

When the composition comprises 7.0% by mass of the ultraviolet scattering agent particles, both the compositions of Comparative Examples 1 and 2 exhibited only a slight improvement in ultraviolet absorption ability as compared to the composition of Reference Example 1. In this case, the ultraviolet absorption ability of the composition of Comparative Example 1, which used polyhydroxystearic acid, which is one of the specific dispersants of the present invention, in addition to another dispersant (PEG-9 polydimethylsiloxyethyl dimethicone) as the dispersant, and the ultraviolet absorption ability of the composition of Comparative Example 2, which used isostearic acid, which is one of the specific dispersants of the present invention, in addition to another dispersant (PEG-9 polydimethylsiloxyethyl dimethicone) as the dispersant, were comparable to each other. Specifically, it is understood that even when the composition comprises 7.0% by mass of ultraviolet scattering agent particles and also comprises the specific dispersant of the present invention, regarding the integrated value of absorbance in the entirety of the UVA and UVB regions, the ultraviolet absorption ability of the compound I is comparable to that of octocrylene, which is a conventional ultraviolet absorber.

When the content of the ultraviolet scattering agent particles was further increased to 10% by mass in the composition, the compositions of Examples 1 and 2, in which the compound I was used, both had higher boost rates (i.e., had significantly improved ultraviolet absorption ability in the entirety of the UVA and UVB regions) as compared to the compositions of Comparative Examples 3 and 4, in which octocrylene was used. From this, it is understood that when there is a relatively large amount of ultraviolet scattering agent particles in the composition (for example, more than 7.0% by mass), by containing the specific dispersant of the present invention and using the compound I in place of octocrylene, the ultraviolet absorption ability is significantly improved.

Furthermore, the composition of Example 1, in which polyhydroxystearic acid was used as the specific dispersant of the present invention, had a high boost rate (i.e., had significantly improved ultraviolet absorption ability in the entirety of the UVA and UVB regions) as compared to the case of Example 2, in which isostearic acid was used as the specific dispersant of the present invention. From this, it is understood that when the content of the ultraviolet scattering agent particles in the composition is equal to or greater than a certain level, a synergistic effect between the compound I and the specific dispersant of the present invention (for example, polyhydroxystearic acid) occurs, and as a result, the ultraviolet absorption ability of the composition is significantly improved.

Regarding the compositions of Comparative Examples 3 and 4 which used octocrylene, boost rates thereof were comparable to each other for the case in which polyhydroxystearic acid was used as the specific dispersant of the present invention in addition to another dispersant (PEG-9 polydimethylsiloxyethyl dimethicone) (Comparative Example 3) and the case in which isostearic acid was used as the specific dispersant of the present invention in addition to another dispersant (PEG-9 polydimethylsiloxyethyl dimethicone) (Comparative Example 4). From this, it is understood that even if the content of ultraviolet scattering agent particles in the composition is equal to or greater than a certain level, the combination of octocrylene, which is a conventional ultraviolet absorber, and a dispersant does not produce a synergistic effect.

When the content of the ultraviolet scattering agent particles in the composition was further increased to 15% by mass, the compositions of Examples 3 and 4 each had even higher boost rates (i.e., had further significantly improved ultraviolet absorption ability in the entirety of the UVA and UVB regions). This suggests that the greater the content of ultraviolet scattering agent particles in the composition exceeds 7.0% by mass, the greater the ultraviolet absorption ability improvement effect due to the compound I. Furthermore, this suggests that when the amount of ultraviolet scattering agent particles in the composition is relatively large (for example, more than 7.0% by mass), a synergistic effect occurs due to the combination of the compound I and the specific dispersant.

### <Comparative Examples 5 and 6>

Compositions of Comparative Examples 5 and 6 were prepared based on the compositions (% by mass) of Table 1 below.

The ultraviolet absorption wavelength of each prepared composition was measured, and the integrated value of absorbance at 290 to 400 nm (the entirety of the UVA and UVB regions) was determined. Using Comparative Example 5 as a reference, the boost rates of Comparative Example 3, Example 1, and Comparative Example 6 were determined, and the results are shown in Table 2. Note that the details of the components of "others" of Table 2 are the same as in Table 1 described above.

**[Table 2]**

| (Table 2: Example 1 and Comparative Examples 3, 5, and 6) | | | | | |
|---|---|---|---|---|---|
| Composition | | Comparative Example 5 | Comparative Example 3 | Example 1 | Comparative Example 6 |
| Water | Water | 34.7 | 33.7 | 33.7 | 34.7 |
| Specific dispersant of present invention | Isostearic acid | - | - | - | - |
| | Polyhydroxys tearic acid | 1.0 | 1.0 | 1.0 | - |
| Another dispersant | PEG-9 polydimethyl siloxyethyl dimethicone | 2.0 | 2.0 | 2.0 | 2.0 |
| UV absorber | Compound I | - | - | 1.0 | 1.0 |
| | Octocrylene | - | 1.0 | - | - |
| UV scattering agent particles | Fatty acid-treated titanium oxide | 5.0 | 5.0 | 5.0 | 5.0 |
| | Fatty acid-treated zinc oxide | 5.0 | 5.0 | 5.0 | 5.0 |
| Others | Others | 54.3 | 54.3 | 54.3 | 54.3 |
| Total | | 100 | 100 | 100 | 100 |
| Evaluation | Boost rate | Reference | 121% | 143% | 101% |

As is clear from Table 2, although the content of the ultraviolet scattering agent particles in the composition was 10% by mass, since the composition of Comparative Example 6, in which the compound I was used, did not contain a dispersant, the boost rate thereof was low. From this, it is understood that in order to improve the ultraviolet absorption ability of a composition when the content of ultraviolet scattering agent particles in the composition is equal to or greater than a certain level, it is necessary to use the compound I and a dispersant together.

### <Examples 5 to 7 and Comparative Examples 7 to 13>

The compositions of Examples 5 to 7 and Comparative Examples 7 to 13 were prepared based on the compositions of Tables 3 and 4 below. Note that the compositions of Comparative Examples 8 to 13, which did not contain the "compound I" and were compensated with an increased amount of water, were otherwise the same as the compositions of Examples 2, 5, 6, 1, and 7 and Comparative Example 7, respectively.

The ultraviolet absorption ability improvement effect of the compositions of each of Examples 2, 5, 6, 1, and 7 and Comparative Example 7 was determined as a "boost rate." The results are shown in Table 3. Note that when calculating the boost rate, Comparative Examples 8 to 13 were used as references for Examples 2, 5, 6, 1, and 7, and Comparative Example 7, respectively.

**[Table 3]**

| (Table 3: Examples 2, 5, 6, 1, and 7, and Comparative Example 7) | | | | | | | |
|---|---|---|---|---|---|---|---|
| Composition | | Example 2 | Example 5 | Example 6 | Example 1 | Example 7 | Comparative Example 7 |
| Water | Water | 33.7 | 33.7 | 33.7 | 33.7 | 33.7 | 33.7 |
| Specific dispersant of present invention | Isostearic acid | 1.0 | - | - | - | - | - |
| | Sorbitan sesquiisostearate | - | 1.0 | - | - | - | - |
| | Bisbutyl dimethicone polyglyceryl-3 | - | - | 1.0 | - | - | - |
| | Polyhydroxystearic acid | - | - | - | 1.0 | - | - |
| | Polyglyceryl-6 polyricinoleate | - | - | - | - | 1.0 | - |
| Another dispersant | PEG-9 polydimethylsiloxyethyl dimethicone | 2.0 | 2.0 | 2.0 | 2.0 | 2.0 | 3.0 |
| UV absorber | Compound I | 1.0 | 1.0 | 1.0 | 1.0 | 1.0 | 1.0 |
| | Octocrylene | - | - | - | - | - | - |
| UV scattering agent | Fatty acid-treated titanium oxide | 5.0 | 5.0 | 5.0 | 5.0 | 5.0 | 5.0 |
| | Fatty acid-treated zinc oxide | 5.0 | 5.0 | 5.0 | 5.0 | 5.0 | 5.0 |
| Others | Others | 54.3 | 54.3 | 54.3 | 54.3 | 54.3 | 54.3 |
| Total | | 100 | 100 | 100 | 100 | 100 | 100 |
| Evaluation | Boost rate | 131% | 132% | 140% | 143% | 139% | 118% |
| | Boost rate calculation reference | Comparative Example 8 | Comparative Example 9 | Comparative Example 10 | Comparative Example 11 | Comparative Example 12 | Comparative Example 13 |

**[Table 4]**

| (Table 4: Comparative Examples 8 to 13) | | | | | | | |
|---|---|---|---|---|---|---|---|
| Composition | | Comparative Example 8 | Comparative Example 9 | Comparative Example 10 | Comparative Example 11 | Comparative Example 12 | Comparative Example 13 |
| Water | Water | 34.7 | 34.7 | 34.7 | 34.7 | 34.7 | 34.7 |
| Specific dispersant of present invention | Isostearic acid | 1.0 | - | - | - | - | - |
| | Sorbitan sesquiisostearate | - | 1.0 | - | - | - | - |
| | Bisbutyl dimethicone polyglyceryl-3 | - | - | 1.0 | - | - | - |
| | Polyhydroxystearic acid | - | - | - | 1.0 | - | - |
| | Polyglyceryl-6 polyricinoleate | - | - | - | - | 1.0 | - |
| Another dispersant | PEG-9 polydimethylsiloxyethyl dimethicone | 2.0 | 2.0 | 2.0 | 2.0 | 2.0 | 3.0 |
| UV absorber | Compound I | - | - | - | - | - | - |
| | Octocrylene | - | - | - | - | - | - |
| UV scattering agent | Fatty acid-treated titanium oxide | 5.0 | 5.0 | 5.0 | 5.0 | 5.0 | 5.0 |
| | Fatty acid-treated zinc oxide | 5.0 | 5.0 | 5.0 | 5.0 | 5.0 | 5.0 |
| Others | Others | 54.3 | 54.3 | 54.3 | 54.3 | 54.3 | 54.3 |
| Total | | 100 | 100 | 100 | 100 | 100 | 100 |

The details of the components of "others" of Tables 3 and 4 are the same as in Table 1 described above.

As is clear from Table 3, the compositions of Examples 2, 5, 6, 1, and 7, which contained the compound I, all had high boost rates (i.e., had significantly improved ultraviolet absorption ability in the entirety of the UVA and UVB regions) as compared to Comparative Examples 8 to 12, which did not contain the compound I.

Furthermore, it was found that the compositions of Examples 2, 5, 6, 1, and 7, which contained different types of dispersants, had different effects on improving the ultraviolet absorption ability depending on the type of dispersant. Among these, in particular, the compositions of Examples 2, 5, 6, 1, and 7, in which isostearic acid, sorbitan sesquiisostearate, bisbutyl dimethicone polyglyceryl-3, polyhydroxystearic acid, and polyglyceryl-6 polyricinoleate, respectively, were used as the dispersant, had higher boost rates (i.e., had significantly improved ultraviolet absorption ability in the entirety of the UVA and UVB regions).

### <<Preparation of Oil-Based Cosmetic Compositions

### <Examples 8 to 12 and Comparative Examples 14 to 20 >

The compositions of Examples 8 to 12 and Comparative Examples 13 to 20 were prepared based on the compositions (% by mass) of Tables 5 and 6 below. The compositions of Comparative Examples 15 to 20, which did not contain the "compound I" and were compensated with an increased amount of the oil content, were otherwise the same as the compositions of Examples 8 to 12 and Comparative Example 14, respectively.

The ultraviolet absorption ability improvement effect of each of the compositions of Examples 8 to 13 and Comparative Example 15 was determined as a "boost rate." The results are shown in Table 5. Note that when calculating the boost rate, Comparative Examples 15 to 20 were used as references for Examples 8 to 13 and Comparative Example 15, respectively.

**[Table 5]**

| Table 5: Examples 8 to 12 and Comparative Example 14) | | | | | | | |
|---|---|---|---|---|---|---|---|
| Composition | | Example 8 | Example 9 | Example 10 | Example 1 | Example 12 | Comparative Example 14 |
| Oil content | Diisopropyl sebacate | 27 | 27 | 27 | 27 | 27 | 27 |
| | Hydrogenated polydecene | 20 | 20 | 20 | 20 | 20 | 20 |
| | Diphenylsiloxyphenyl trimethicone | 20 | 20 | 20 | 20 | 20 | 20 |
| UV absorber | Compound I | 1.0 | 1.0 | 1.0 | 1.0 | 1.0 | 1.0 |
| UV scattering agent | Fatty acid-treated titanium oxide | 5.0 | 5.0 | 5.0 | 5.0 | 5.0 | 5.0 |
| | Fatty acid-treated zinc oxide | 5.0 | 5.0 | 5.0 | 5.0 | 5.0 | 5.0 |
| Specific dispersant of present invention | Isostearic acid | 1.0 | - | - | - | - | - |
| | Sorbitan sesquiisostearate | - | 1.0 | - | - | - | - |
| | Bisbutyl dimethicone polyglyceryl-3 | - | - | 1.0 | - | - | - |
| | Polyhydroxystearic acid | - | - | - | 1.0 | - | - |
| | Polyglyceryl-6 polyricinoleate | - | - | - | - | 1.0 | - |
| Oher dispersant | PEG-9 polydimethylsiloxyethyl dimethicone | - | - | - | - | - | 1.0 |
| Others | Others | 21 | 21 | 21 | 21 | 21 | 21 |
| Total | | 100 | 100 | 100 | 100 | 100 | 100 |
| Evaluation | Boost rate | 129% | 127% | 136% | 139% | 142% | 115% |
| | Boost rate calculation reference | Comparative Example 15 | Comparative Example 16 | Comparative Example 17 | Comparative Example 18 | Comparative Example 19 | Comparative Example 20 |

**[Table 6]**

| (Table 6: Comparative Example 15 to 20) | | | | | | | |
|---|---|---|---|---|---|---|---|
| Composition | | Comparative Example 15 | Comparative Example 16 | Comparative Example 17 | Comparative Example 18 | Comparative Example 19 | Comparative Example 20 |
| Oil content | Diisopropyl sebacate | 28 | 28 | 28 | 28 | 28 | 28 |
| | Hydrogenated polydecene | 20 | 20 | 20 | 20 | 20 | 20 |
| | Diphenylsiloxyphenyl trimethicone | 20 | 20 | 20 | 20 | 20 | 20 |
| UV absorber | Compound I | - | - | - | - | - | - |
| UV scattering agent | Fatty acid-treated titanium oxide | 5.0 | 5.0 | 5.0 | 5.0 | 5.0 | 5.0 |
| | Fatty acid-treated zinc oxide | 5.0 | 5.0 | 5.0 | 5.0 | 5.0 | 5.0 |
| Specific dispersant of present invention | Isostearic acid | 1.0 | - | - | - | - | - |
| | Sorbitan sesquiisostearate | - | 1.0 | - | - | - | - |
| | Bisbutyl dimethicone polyglyceryl-3 | - | - | 1.0 | - | - | - |
| | Polyhydroxystearic acid | - | - | - | 1.0 | - | - |
| | Polyglyceryl-6 polyricinoleate | - | - | - | - | 1.0 | - |
| Another dispersant | PEG-9 polydimethylsiloxyethyl dimethicone | - | - | - | - | - | 1.0 |
| Others | Others | 21 | 21 | 21 | 21 | 21 | 21 |
| Total | | 100 | 100 | 100 | 100 | 100 | 100 |

The details of the components of "others" of Tables 5 and 6 are as follows:

| | |
|---|---|
| ·Ethanol | 5.0% by mass |
| ·Glycerin | 1.0 % by mass |
| ·Hydroxystearic acid | 6.0 % by mass |
| ·Polyamide-8, pentaerythrityl tetra(di-t-butylhydroxyhydrocinnamate) | 2.0 % by mass |
| ·Dibutyl lauroyl glutamide | 2.0 % by mass |
| ·Silica powder | 5.0 % by mass |

As is clear from Table 5, all of the compositions of Examples 8 to 12, which contained the compound I, had large boost rates (i.e., the ultraviolet absorption ability in the entirety of the UVA and UVB regions was greatly improved), as compared to Comparative Examples 15 to 19, which did not contain the compound I.

Furthermore, it was found that the compositions of Examples 8 to 12, which contained different types of dispersants, had different ultraviolet absorption ability improvement effects depending on the type of dispersant. Among these, in particular, the compositions of Examples 8 to 12, in which isostearic acid, sorbitan sesquiisostearate, bisbutyl dimethicone polyglyceryl-3, polyhydroxystearic acid, and polyglyceryl-6 polyricinoleate were used as dispersants, respectively, had higher boost rates (i.e., had significantly improved ultraviolet absorption ability in the entirety of the UVA and UVB regions).

### <<Preparation of Oil-in-Water Cosmetic Compositions

### <Examples 13 to 17 and Comparative Examples 21 to 27>

The compositions of Examples 13 to 17 and Comparative Examples 21 to 27 were prepared based on the compositions (% by mass) of Table 7 and 8 below. The compositions of Comparative Examples 22 to 27, which did not contain the "compound I" and were compensated with an increased amount of water, were otherwise the same as the compositions of Examples 13 to 17 and Comparative Example 21, respectively.

The ultraviolet absorption ability improvement effect of each of the compositions of Examples 13 to 17 and Comparative Example 21 was determined as a "boost rate." The results are shown in Table 7. Note that when calculating the boost rate, Comparative Examples 22 to 27 were used as references for Examples 13 to 17 and Comparative Example 21, respectively.

**[Table 7]**

| (Table 7: Examples 13 to 17 and Comparative Example 21) | | | | | | | |
|---|---|---|---|---|---|---|---|
| Composition | | Example 13 | Example 14 | Example 15 | Example 16 | Example 17 | Comparative Example 21 |
| Water | Water | Balance | Balance | Balance | Balance | Balance | Balance |
| UV absorber | Compound I | 1.0 | 1.0 | 1.0 | 1.0 | 1.0 | 1.0 |
| UV scattering agent | Fatty acid-treated titanium oxide | 5.0 | 5.0 | 5.0 | 5.0 | 5.0 | 5.0 |
| | Fatty acid-treated zinc oxide | 5.0 | 5.0 | 5.0 | 5.0 | 5.0 | 5.0 |
| Specific dispersant of present invention | Isostearic acid | 1.0 | - | - | - | - | - |
| | Sorbitan sesquiisostearate | - | 1.0 | - | - | - | - |
| | Bisbutyl dimethicone polyglyceryl-3 | - | - | 1.0 | - | - | - |
| | Polyhydroxystearic acid | - | - | - | 1.0 | - | - |
| | Polyglyceryl-6 polyricinoleate | - | - | - | - | 1.0 | - |
| Another dispersant | PEG-9 polydimethylsiloxyethyl dimethicone | - | - | - | - | - | 1.0 |
| | PEG-60 hydrogenated castor oil | 1.6 | 1.6 | 1.6 | 1.6 | 1.6 | 1.6 |
| Buffer | Buffer | Appropriate amount | Appropriate amount | Appropriate amount | Appropriate amount | Appropriate amount | Appropriate amount |
| Preservative | Phenoxyethanol | Appropriate amount | Appropriate amount | Appropriate amount | Appropriate amount | Appropriate amount | Appropriate amount |
| Others | Others | 45.32 | 45.32 | 45.32 | 45.32 | 45.32 | 45.32 |
| Total | | 100 | 100 | 100 | 100 | 100 | 100 |
| Evaluation | Boost rate | 124% | 125% | 136% | 138% | 139% | 117% |
| | Boost rate calculation reference | Comparative Example 22 | Comparative Example 23 | Comparative Example 24 | Comparative Example 25 | Comparative Example 26 | Comparative Example 27 |

**[Table 8]**

| (Table 8: Comparative Examples 22 to 27) | | | | | | | |
|---|---|---|---|---|---|---|---|
| Composition | | Comparative Example 22 | Comparative Example 23 | Comparative Example 24 | Comparative Example 25 | Comparative Example 26 | Comparative Example 27 |
| Water | Water | Balance | Balance | Balance | Balance | Balance | Balance |
| UV absorber | Compound I | - | - | - | - | - | - |
| UV scattering agent | Fatty acid-treated titanium oxide | 5.0 | 5.0 | 5.0 | 5.0 | 5.0 | 5.0 |
| | Fatty acid-treated zinc oxide | 5.0 | 5.0 | 5.0 | 5.0 | 5.0 | 5.0 |
| Specific dispersant of present invention | Isostearic acid | 1.0 | - | - | - | - | - |
| | Sorbitan sesquiisostearate | - | 1.0 | - | - | - | - |
| | Bisbutyl dimethicone polyglyceryl-3 | - | - | 1.0 | - | - | - |
| | Polyhydroxystearic acid | - | - | - | 1.0 | - | - |
| | Polyglyceryl-6 polyricinoleate | - | - | - | - | 1.0 | - |
| Another dispersant | PEG-9 polydimethylsiloxyethyl dimethicone | - | - | - | - | - | 1.0 |
| | PEG-60 hydrogenated castor oil | 1.6 | 1.6 | 1.6 | 1.6 | 1.6 | 1.6 |
| Buffer | Buffer | Appropriate amount | Appropriate amount | Appropriate amount | Appropriate amount | Appropriate amount | Appropriate amount |
| Preservative | Phenoxyethanol | Appropriate amount | Appropriate amount | Appropriate amount | Appropriate amount | Appropriate amount | Appropriate amount |
| Others | Others | 45.32 | 45.32 | 45.32 | 45.32 | 45.32 | 45.32 |
| Total | | 100 | 100 | 100 | 100 | 100 | 100 |

The details of the components of "others" of Tables 7 and 8 are as follows:

| | |
|---|---|
| ·Glycerin | 4.0% by mass |
| ·1,3-Butylene glycol | 7.0% by mass |
| ·Succinoglycan | 0.12% by mass |
| ·Sucrose fatty acid | 3.0% by mass |
| ester·(Dimethylacrylamide/acryloyldimethyltaurine Na) crosspolymer | 0.6% by mass |
| ·Diisopropyl sebacate | 4.0% by mass |
| ·Nonvolatile dimethicone | 2.0% by mass |
| ·PPG-17 | 1.0% by mass |
| ·Cyclomethicone | 12% by mass |
| ·Triethylhexanoin | 5.0% by mass |
| ·Silica powder | 5.0% by mass |

The details of "buffer" of Tables 7 and 8 are as follows:
Citric acid: Appropriate amount
Sodium citrate: Appropriate amount
EDTA-3Na: Appropriate amount

As is clear from Table 7, Examples 13 to 17, which contained the compound I, all had high boost rates (i.e., the ultraviolet absorption ability in the entirety of the UVA and UVB regions was greatly improved) as compared to Comparative Examples 20 to 25, which did not contain the compound I.

Furthermore, it was found that the compositions of Examples 13 to 17, which contained different types of dispersants, had different ultraviolet absorption ability improvement effects depending on the type of dispersant. Among these, in particular, the compositions of Examples 13 to 17, in which isostearic acid, sorbitan sesquiisostearate, bisbutyl dimethicone polyglyceryl-3, polyhydroxystearic acid, and polyglyceryl-6 polyricinoleate were used as the dispersant, respectively, had significantly higher boost rates (i.e., the ultraviolet absorption ability in the entirety of the UVA and UVB regions was greatly improved).

## Claims

1. A cosmetic composition, comprising components (i) to (iii) below, and an oil content which is capable of at least partially dissolving the component (i):
(i) a compound I having a structure of formula (I) below: 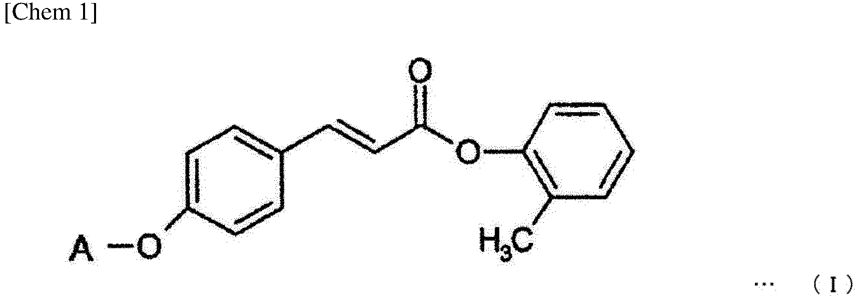 where -OA represents an alkoxy group,
(ii) ultraviolet scattering agent particles, and
(iii) a dispersant,
wherein a content of the ultraviolet scattering agent particles is more than 7.0% by mass, and
wherein the dispersant contains at least one dispersant selected from the group consisting of polyhydroxystearic acid, isostearic acid, a polyglyceryl-based dispersant, and a sorbitan-based dispersant.

2. The composition according to claim 1, wherein the content of the ultraviolet scattering agent particles is 10% by mass or more.

3. The composition according to claim 1 or 2, wherein the dispersant contains at least one dispersant selected from the group consisting of the polyhydroxystearic acid and the polyglyceryl-based dispersant.

4. The composition according to any one of claims 1 to 3, wherein the polyglyceryl-based dispersant is a polyglycerin-modified silicone or a fatty acid polyglyceryl ester having 3 to 8 glycerin units.

5. The composition according to any one of claims 1 to 4, wherein a content of the dispersant is 0.5% by mass or more and 3.5% by mass or less.

6. The composition according to any one of claims 1 to 5, wherein the oil content contains a polar oil.

7. The composition according to any one of claims 1 to 6, which is an oil-in-water cosmetic.

8. The composition according to any one of claims 1 to 6, which is a water-in-oil cosmetic.

9. The composition according to any one of claims 1 to 6, which is an oil-based cosmetic.

10. The composition according to any one of claims 1 to 9, which is a sunscreen cosmetic composition.
